# EUROPEAN PATENT SPECIFICATION

(11) **EP 0 496 630 B1**
(45) Date of publication and mention of the grant of the patent: **07.06.1995**
(21) Application number: 92300607.6
(22) Date of filing: 24.01.1992
(51) Int. Cl.: C07C 317/44, C07C 317/46, C07C 323/62, A01N 41/10

(54) **2-Cyano-1,3-dione herbicides**
2-Cyano-1,3-dionherbizide
Herbicides à base de 2-cyano-1,3-dione

(30) Priority: 25.01.1991 GB 9101659
(43) Date of publication of application: 29.07.1992
(73) Proprietor: RHONE-POULENC AGRICULTURE LTD., Ongar, Essex CM5 0HW (GB)
(72) Inventor: Cain, Paul Alfred c/o Research Station, Ongar, Essex CM5 OHW (GB); Cramp, Susan Mary c/o Research Station, Ongar, Essex CM5 OHW (GB)
(74) Representative: Bentham, Stephen

(56) References cited:
- EP-A- 0 213 892
- EP-A- 0 374 811
- EP-A- 0 398 692

## Description

This invention relates to novel 2-cyano-1,3-dione derivatives, processes for their preparation, compositions containing them, and their use as herbicides.

The present invention provides 2-cyano-1,3-diones of general formula I: 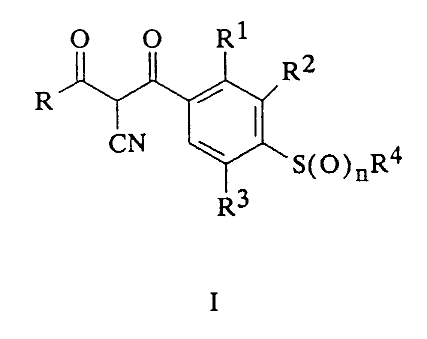
wherein:
R represents:-
a straight- or branched-chain alkyl group containing up to 6 carbon atoms which is optionally substituted by one or more halogen atoms which may be the same or different; or
a cycloalkyl group containing from 3 to 6 carbon atoms which is optionally substituted by one or more groups selected from R⁵ and one or more halogen atoms which may be the same or different;
R¹ represents:-
a halogen atom, or
a straight- or branched-chain alkyl group containing up to 6 carbon atoms which is substituted by -OR⁵; or
a group selected from R⁵, cyano, -SR⁵, -OR⁵, -O(CH₂)ₘOR⁵, or -CO₂R⁵;
R² and R³, which may be the same or different, each represents:-
a halogen or hydrogen atom, or
a straight- or branched-chain alkyl group containing up to 6 carbon atoms which is substituted by -OR⁵; or
a group selected from R⁵, nitro, cyano, -OR⁵, -O(CH₂)ₘOR⁵ or -CO₂R⁵;
provided that at least one of R² and R³ is hydrogen;
R⁴ and R⁵, which may be the same or different, each represents:-
a straight- or branched-chain alkyl group containing up to 6 carbon atoms which is optionally substituted by one or more halogen atoms which may be the same or different;
n is zero, 1 or 2; and m is an integer from 1 to 3;
and where the compounds exist in enolic tautomeric forms, agriculturally acceptable salts thereof, which possess valuable herbicidal properties.

Compounds of formula I may exist in enolic tautomeric forms that may give rise to geometric isomers around the enolic double bond. Furthermore, in certain cases the substituents R, R¹, R², R³, R⁴, and R⁵ may contribute to optical isomerism and/or stereoisomerism. All such forms are embraced by the present invention.

By the term "agriculturally acceptable salts" is meant salts the cations of which are known and accepted in the art for the formation of salts for agricultural or horticultural use. Preferably the salts are water soluble.

Suitable salts formed by compounds of general formula I which are acidic, i.e. in enolic tautomeric forms, with bases include alkali metal (e.g. sodium and potassium) salts, alkaline earth metal (e.g. calcium and magnesium) salts, ammonium (e.g. dioctylmethylamine and morpholine) salts.

The compounds of this invention represent in some aspects of their activivity an improvement over known compounds.

Particularly important classes of compounds of formula I because of their herbicidal properties are those containing one or more of the following features:-
(a) R represents for example ethyl, methyl, n-propyl, isopropyl, t-butyl, cyclopropyl or 1-methylcyclopropyl; and/or
(b) R¹ represents:-
   a halogen atom; or
   a group selected from
   -OR⁵, for example methoxy, ethoxy or trifluoromethoxy; or
   R⁵, for example methyl or trifluoromethyl; and/or
(c) R² and R³, which may be the same or different, each represents:-
   a halogen or hydrogen atom; or
   a straight- or branched-chain alkyl group containing up to 6 carbon atoms which is substituted by -OR⁵, for example methoxymethyl; or
   a group selected from
   R⁵, for example methyl, or trifluoromethyl; or
   -OR⁵, for example methoxy, ethoxy or isopropoxy,
   -O(CH₂)ₘOR⁵ where m is 2 or 3, for example 2-ethoxyethoxy or 2-methoxyethoxy,
   or -CO₂R⁵, for example carboethoxy, carbomethoxy, or carboisopropoxy,
   provided that at least one of R² and R³ is hydrogen; and/or
(d) R⁴ represents:-
   a straight- or branched-chain alkyl group containing up to 4 carbon atoms which is optionally substituted by one or more halogen atoms which may be the same or different, for example methyl, ethyl or trifluoromethyl; and/or
(e) R⁵ represents:-
   a straight- or branched-chain alkyl group containing up to 4 carbon atoms which is optionally substituted by one or more halogen atoms which may be the same or different, for example methyl, isopropyl, or trifluoromethyl; and
(f) 'halogen' means chlorine, bromine or fluorine.

A further preferred class of compounds of general formula I are those in which R³ represents a hydrogen atom.

A further preferred class of compounds of general formula I are those wherein:
R¹ represents a group selected from R⁵, cyano, -SR⁵, -O(CH₂)ₘOR⁵ and -CO₂R⁵; preferably R⁵; and
R⁵ represents a straight- or branched-chain alkyl group containing up to 6 carbon atoms.

A further preferred class of compounds of general formula I are those wherein:
R represents methyl, ethyl, isopropyl, t-butyl, cyclopropyl or 1-methylcyclopropyl;
R¹ represents chlorine, bromine, fluorine, trifluoromethyl or methoxy;
R² represents hydrogen, chlorine or methoxy;
R³ represents hydrogen;
R⁴ represents methyl or ethyl; and
n is 0, 1 or 2.

The compounds of general formula I can be prepared as hereinafter described. In the following description where symbols appearing in formulae are not specifically defined it is to be understood that they are as hereinbefore defined in accordance with the first definition of each symbol in this specification.

It is to be understood that in the description of the following processes the sequences may be performed in different orders and that suitable protecting groups may be required to achieve the compounds sought.

According to a feature of the present invention the compounds of general formula I where n is 0 or 2 may be prepared by the reaction of a beta-ketonitrile of formula II with a benzoyl chloride of formula III where n is 0 or 2 accordidng to the following reaction scheme:

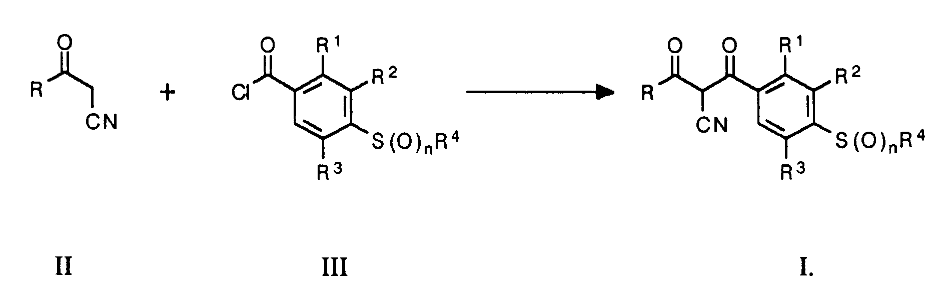

According to a further feature of the present invention, compounds of general formula I where n is 0 or 2 may be prepared by the reaction of a beta-ketonitrile of formula V where n is 0 or 2 with an acid chloride of formula IV according to the following reaction scheme: 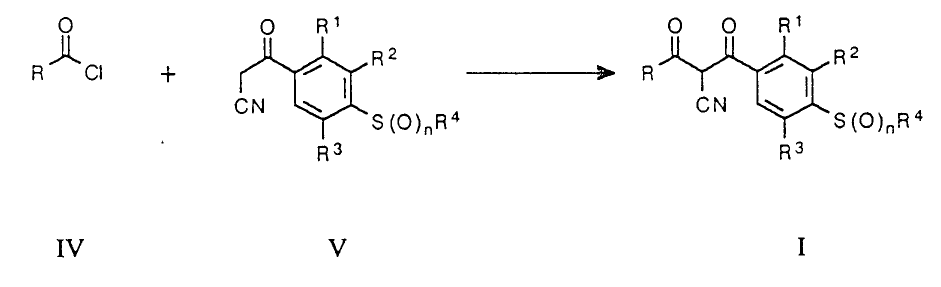
In general, these reactions are performed under the influence of a base in a solvent or solvent mixture. Suitable bases include metal (e.g. alkali metal or alkaline earth metal, preferably alkali metal) hydrides, hydroxides or alkoxides or salts such as sodium or lithium hydride, sodium hydroxide, potassium hydroxide, or magnesium ethoxide or magnesium methoxide, or potassium carbonate; and organic bases such as triethylamine. Suitable solvents in which to conduct these reactions include ethers, such as tetrahydrofuran; hydrocarbons, such as toluene; or halogenated hydrocarbons, such as dichloromethane. These reactions can be conducted at a temperature between 0° C and the boiling point of the solvent.

Intermediates in the preparation of compounds of general formula I may be prepared by the application or adaptation of known methods. For instance, beta-ketonitriles of formula II may be prepared from acid chlorides of general formula IV by a number of methods well known in the chemical literature. For example, see Krauss, et al, *Synthesis*, 1983, 308 or Muth, et al, *J. Org. Chem*., 1960**, 25**, 736. Alternatively, esters of general formula VI may be reacted with acetonitrile in accordance with Abramovitch and Hauser, *J. Am. Chem. Soc*., 1942, **64**, 2720, to provide beta-ketonitriles of formula II according to the following reaction scheme:

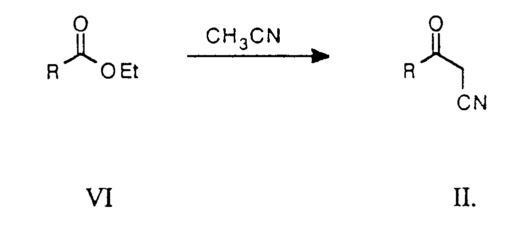

The intermediate beta-ketonitriles of formula V where n is 0 may be prepared from benzoyl chlorides III where n is 0 or from ethyl benzoates of general formula VII where n is 0 in a manner analogous to the preparation of beta-ketonitriles of formula II set forth above:

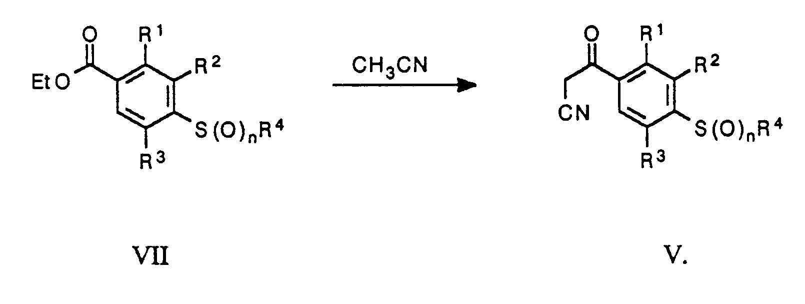

Intermediate acid chlorides of general formula III or IV are generally known or can be prepared from the corresponding carboxylic acid according to commonly accepted methods, for example by treatment with thionyl chloride in chloroform at reflux.

Interconversion of compounds of general formula I or of intermediates is possible by the application or adaptation of known methods. Compounds in which n is 1 or 2 may be prepared by oxidation of the compounds in which n is 0 using, for example, 3-chloroperoxybenzoic acid in an inert solvent such as dichloromethane at a temperature between -30° C and the boiling point of the solvent.

Compounds in which R¹, R² or R³ is a halogen atom may be prepared from corresponding compounds in which R¹, R², or R³ is replaced by an unsubstituted amino group by a Sandmeyer type reaction. This may be carried out using sodium nitrite in the presence of an acid such as hydrochloric acid or hydrobromic acid followed by treatment with for example copper (I) chloride or copper (I) bromide between room temperature and 80° C. Alternatively, diazotization may be carried out using an alkyl nitrite such as t-butyl nitrite in the presence of a halogenating agent such as copper (II) chloride or bromoform in an inert solvent such as acetonitrile.

Esters of general formula VII where n is 0 may be prepared by treatment of compounds of the general formula VIII in which X is a nitro group or a chlorine or bromine atom with an alkylmercaptan (i.e. R⁴SH) in the presence of a base in an inert solvent: 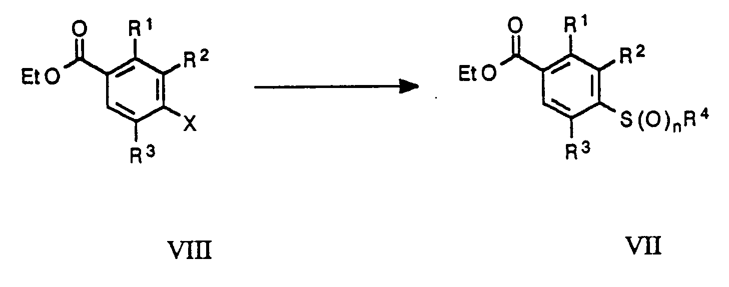
Suitable bases include sodium hydroxide and potassium carbonate; suitable inert solvents include dimethylformamide and dimethyl sulphoxide. The reaction may be conducted at a temperature between room temperature and 150° C.

Esters of general formula VII where n is 0 may also be prepared from compounds of general formula VIIIa: 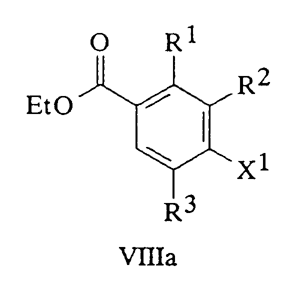
in which X¹ is an unsubstituted amino group by the action of an alkyl nitrite such as tert-butylnitrite and a dialkyl disulfide in the presence of an inert solvent, for example chloroform at a temperature from ambient to the boiling point of the solvent.

Compounds in which R¹, R² or R³ is replaced by an unsubstituted amino group may be prepared by the reduction of compounds in which R¹, R² or R³ represents a nitro group, for example by means of tin (II) chloride and hydrochloric acid.

Compounds in which R¹, R² or R³ represents a cyano group may be prepared from compounds in which R¹, R² or R³ represents a group CO₂R⁵ via hydrolysis to the corresponding carboxylic acid, in which R⁵ is replaced by hydrogen, conversion to a corresponding acid halide, for example by treatment with thionyl chloride, treatment with ammonia to give the amide, and dehydration, for example by means of phosphorus oxychloride. Compounds in which R¹, R² or R³ represents a nitro group may be prepared by the oxidation of corresponding compounds in which R¹, R² or R³ is replaced by an unsubstituted amino group, for example by means of reaction with trifluoroperacetic acid.
The following example illustrates the invention.

### EXAMPLE 1: Preparation of 1-[2-chloro-4-(methylsulphonyl)phenyl]-2-cyano-4,4-dimethylpentan-1,3-dione, Compound A.

To a mixture of absolute ethanol (10 ml) and carbon tetrachloride (1 ml) under an inert atmosphere and at room temperature was added magnesium turnings (0.2g). The reaction mixture was stirred; almost immediately a reaction started (effervescence observed). To the reaction mixture was added 4,4-dimethyl-3-oxopentanonitrile (1g) in absolute ethanol (4 ml) and the resulting suspension gently warmed until all the magnesium had reacted (approximately 30 min.). The resultant yellow solution was evaporated to dryness, dry toluene (30 ml) was added and the solvents evaporated. The solid was suspended in dry toluene (20 ml) and the resulting mixture warmed to 50° C. A slurry of 2-chloro-4-(methylsulphonyl)benzoyl chloride in dry toluene (10 ml) was added. The reaction mixture was heated at reflux for 1 hour, cooled to room temperature and allowed to sit overnight. To the reaction mixture was added 6N HCl (70 ml) and the two layers stirred vigorously until all the solid had dissolved. The layers were separated and the aqueous layer was extracted with ether (twice with 30 ml portions). The organic extracts were combined with the organic layer and extracted with saturated NaHCO₃ solution (three times with 50 ml portions). The basic extracts were acidified to pH 6 by the careful addition of concentrated HCl and the resultant solution was extracted with ether. The ethereal extracts were combined, washed with water and dried (MgSO₄). After removal of the drying agent by filtration, evaporation of the solvents afforded 0.85g of a light brown solid. This material was suspended in boiling cyclohexane and enough ethyl acetate was added to dissolve the solid. After cooling to room temperature the precipitate was recovered by filtration and dried to afford 0.6 g of 1-[2-chloro-4-(methylsulphonyl)phenyl]-2-cyano-4,4-dimethylpentan-1,3-dione, Compound A, as a light brown solid, m.p. 144°C.

The compounds listed below may be prepared in manner similar to the method described above.
COMPOUND B: 1-[2-chloro-4-(methylsulphonyl)phenyl]-2-cyano-3-cyclopropylpropan-1,3-dione, m.p. 145° C, starting from 3-cyclopropyl-3-oxo-propanonitrile and 2-chloro-4-(methylsulphonyl)benzoyl chloride.
COMPOUND C: 2-cyano-3-cyclopropyl-1-[4-(methylsulphonyl)-2-trifluoromethylphenyl]propan-1,3-dione, m.p. 139° C, starting from 3-cyclopropyl-3-oxo-propanonitrile and 4-(methylsulphonyl)-2-trifluoromethylbenzoyl chloride
COMPOUND D: 1-[2-chloro-4-(methylsulphonyl)phenyl]-2-cyanopentan-1,3-dione, m.p. 128 °C, starting from 3-oxo-pentanonitrile and 2-chloro-4-(methylsulphonyl)benzoyl chloride.
COMPOUND E: 1-[2-chloro-4-(methylsulphonyl)phenyl]-2-cyano-4-methylhexan-1,3-dione, m.p. 110° C, starting from 3-oxo-4-methylhexanonitrile and 2-chloro-4-(methylsulphonyl)benzoyl chloride.
COMPOUND F: 1-[2-chloro-4-(methylsulphonyl)phenyl]-2-cyanohexan-1,3-dione, m.p. 90° C, starting from 3-oxo-hexanonitrile and 2-chloro-4-(methylsulphonyl)benzoyl chloride.
COMPOUND G: 2-cyano-4,4-dimethyl-1-[4-(methylsulphonyl)-2-trifluoromethylphenyl]pentan-1,3-dione, m.p. 123° C, starting from 4,4-dimethyl-3-oxo-pentanonitrile and 4-(methylsulphonyl)-2-trifluoromethylbenzoyl chloride.
COMPOUND H: 2-cyano-1-[4-(methylsulphonyl)-2-trifluoromethylphenyl]-3-(1-methylcyclopropyl)propan-1,3-dione, m.p. 131° C, starting from 3-(1-methylcyclopropyl)-3-oxopropanonitrile and 4-(methylsulphonyl)-2-trifluoromethylbenzoyl chloride.
COMPOUND I: 2-cyano-1-[4-(methylsulphonyl)-2-trifluoromethylphenyl]butan-1,3-dione, m.p. 150° C, starting from 3-oxo-butanonitrile and 4-(methylsulphonyl)-2-trifluoromethylbenzoyl chloride.
COMPOUND J: 1-[2-chloro-4-(methylsulphenyl) phenyl]-2-cyano-3-cyclopropylpropan-1,3-dione, m.p. 90.5°C, starting from 3-cyclopropyl-3-oxopropanonitrile and 2-chloro-4-(methylsulphenyl) benzoyl chloride.
COMPOUND K: 2-cyano-3-cyclopropyl-1-[2-fluoro-4-(methylsulphonyl)phenyl] propan-1,3-dione, m.p 142°C, starting from 3-cyclopropyl-3-oxopropanonitrile and 2-fluoro-4-(methylsulphonyl) benzoyl chloride.
COMPOUND L: 1-[2-chloro-4-(ethylsulphonyl) phenyl]-2-cyano-3- i cyclopropylpropan-1,3-dione, m.p. 142°C, starting from 3-cyclopropyl -3-oxopropanonitrile and 2-chloro-4-(ethylsulphonyl) benzoyl chloride.
COMPOUND M: 2-cyano-3-cyclopropyl-1-[4-(methylsulphenyl)-2-trifluoromethylphenyl] propan-1,3-dione as a yellow gum, ¹H NMR (CDCl₃): d 1.2 (m,2H), 1.4 (m,2H), 2.3 (m,1H), 2.4 (s,3H), 7.3-7.6 (m,3H), starting from 3-cyclopropyl-3-oxopropanonitrile and 4-(methylsulphenyl)-2-trifluoromethyl benzoyl chloride.
COMPOUND N: 2-cyano-3-cyclopropyl-1-[2-methoxy-4-(methylsulphenyl)phenyl] propan-1,3-dione, m.p. 108°C, starting from 3-cyclopropyl-3-oxopropanonitrile and 2-methoxy-4-(methylsulphenyl)benzoyl chloride.
COMPOUND O: 1-[2-bromo-3-methoxy-4-(methylsulphonyl)phenyl]-2-cyanobutan-1,3-dione, m.p. 111°C, starting from 3-oxobutanonitrile and 2-bromo-3-methoxy-4-(methylsulphonyl)benzoyl chloride.
COMPOUND P: 1-[2,3-dichloro-4-(methylsulphonyl)-phenyl]-2-cyano-3-(1-methylcyclopropyl)propan-1,3-dione, m.p. 179.5°C, starting from 3-(1-methylcyclopropyl)-3-oxopropanonitrile and 2,3-dichloro-4-(methylsulphonyl)benzoyl chloride.
COMPOUND Q: 1-[2-chloro-3-methoxy-4-(methylsulphonyl)phenyl]-2-cyano-3-cyclopropylpropan-1,3-dione, m.p. 147.5°C, starting from 3-cyclopropyl-3-oxopropanonitrile and 2-chloro-3-methoxy-4-(methylsulphonyl)benzoyl chloride.
COMPOUND R: 1-[2-chloro-3-methoxy-4-(methylsulphonyl)phenyl]-2-cyanobutan-1,3-dione, m.p. 123°C, starting from 3-oxobutanonitrile and 2-chloro-3-methoxy-4-(methylsulphonyl)-benzoyl chloride.
COMPOUND S: 1-[2-bromo-4-(methylsulphonyl)-phenyl]-2-cyano-3-cyclopropylpropan-1,3-dione, m.p. 165°C, starting from 3-cyclopropyl-3-oxo-propanonitrile and 2-bromo-4-(methylsulphonyl)benzoyl chloride.
COMPOUND T: 1-[2-bromo-4-(methylsulphonyl)phenyl]-2-cyano-3-(1-methylcyclopropyl)-propan-1,3-dione, m.p. 146°C, starting from 3-(1-methylcyclopropyl)-3-oxopropanonitrile and 2-bromo-4-(methylsulphonyl)benzoyl chloride.
COMPOUND U: 2-cyano-3-(1-methylcyclopropyl)-1-[4-(ethylsulphonyl)-2-trifluoromethylphenyl]propan-1,3-dione as a glass, H¹ NMR (DMSO-d₆): 0.5(m,2H) 0.85(m,2H) 1.3(t,3H) 1.35(s,3H) 3.05(q,2H) 7.1-7.7(m,3H), starting from 3-(1-methylcyclopropyl)-3-oxo-propanonitrile and 4-(ethylsulphenyl)-2-trifluoromethylbenzoyl chloride.
COMPOUND V: 1-[2-chloro-3-methoxy-4-(methylsulphonyl)-2-cyano-3-(1-methylcyclopropyl)propan-1,3-dione, m.p. 127°C, starting from 3-(1-methylcyclopropyl)-3-oxo-propanonitrile and 2-chloro-3-methoxy-4-(methylsulphonyl)benzoyl chloride.

Hereafter follows the procedures for preparation of intermediates relating to the present invention:-

### Example 1(a): Preparation of 4,4-dimethyl-3-oxopentanonitrile.

Cyanoacetic acid (8.5 g) was dissolved in dry THF (300 ml), placed under an inert atmosphere and the solution cooled using a dry ice-acetone bath. Butyl lithium (80 ml of a 2.5M solution in hexane) was added dropwise over 1 hour. During the addition the internal reaction temperature was maintained below -65° C. The reaction mixture was stirred in the dry ice-acetone bath for 1 hour and then the bath was removed and the reaction stirred for an additional hour, during which time the internal temperature rose to -45° C. The resultant reaction mixture was cooled to -70° C and trimethylacetyl chloride (6.0 g) in THF (50 ml) added dropwise over 30 minutes. while keeping the temperature of the reaction mixture below -65° C. The reaction mixture was allowed to stir in a dry ice-acetone bath for 1 hour and then allowed to warm to room temperature overnight. The resultant mixture was acidified by the addition of 2N HCl (200 ml) and diluted with CH₂Cl₂ (500 ml). The layers were separated and the aqueous layer was extracted with CH₂Cl₂. The combined organic layers were dried (MgSO₄) and, after removal of the drying agent by filtration, the solvents were removed. The residue was dissolved in ether (200 ml) and the etheral solution extracted with 2N NaOH (twice with 50 ml portions). To the combined aqueous extracts was added conc. HCl to adjust the solution to pH 1. The resulting mixture was extracted with ether. The combined extracts were dried (MgSO₄) and, after removal of the drying agent by filtration, the solvents were removed to afford 5.6 g of 4,4-dimethyl-3-oxopentanonitrile as an orange oil which slowly solidified; m.p. 60° C.

### Example 2(a)

Cyanoacetic acid (8.5 g) was dissolved in dry THF (250 ml), placed under an inert atmosphere and the solution was cooled using a dry ice-acetone bath. Butyl lithium (80 ml of a 2.5M solution in hexane) was added dropwise over 1 hour. During the addition the internal reaction temperature was maintained below -65° C. The reaction mixture was stirred in the dry ice-acetone bath for 1 hour, the cooling bath removed, and the reaction stirred for an additional hour during which time the internal temperature rose to 15° C. The resultant reaction mixture was cooled to -70° C and cyclopropanecarbonyl chloride (5.2 g) in THF (50 ml) added dropwise over 20 min. while keeping the temperature of the reaction mixture below -65° C. The reaction mixture was thus stirred for 1 hour, and then allowed to warm to room temperature overnight. The resultant mixture was acidified by the addition of 2N HCl (200 ml) and then diluted with CH₂Cl₂ (500 ml). The layers were separated and the aqueous layer was extracted with CH₂Cl₂ . The combined organic layers were dried, filtered; the solvents were removed to give 7.5g of an orange oil. The crude product was chromatographed on silica gel to afford 4.0g of 3-cyclopropyl-3-oxopropanonitrile as a pale orange oil, ¹H-nmr (CDCl₃): d 0.9-1.3 (m, 4H), 1.9-2.1 (m, 1H), 3.66 (s, 2H).

Benzoyl chlorides were prepared by heating the appropriately substituted benzoic acids with thionyl chloride for 3 hours. The excess thionyl choride was removed by evaporation and the benzoyl chlorides were used directly without further purification.

### Example 3(a)

Potassium permanganate (119.2g) was added to a stirred, heated suspension of crude 2-fluoro-4-(methylsulphenyl)toluene (25.37g) in water at approximately 90-100°C. The mixture was cooled slightly and filtered. The solid was washed thoroughly with hot water. The filtrate was extracted with dichloromethane. The aqueous layer was acidified to pH 1 and extracted with ethyl acetate. The organic extract was dried (MgSO₄) and filtered. The filtrate was evaporated to dryness to give 2-fluoro-4-(methylsulphonyl)benzoic acid (15.9g) as an orange solid, m.p. 188°C.

### Example 4(a)

t-Butyl nitrite (4 ml) was added to a mixture of 3-fluoro-4-methylaniline (5 g) and dimethyldisulphide (36 ml) in chloroform. After the reaction had initiated t-butyl nitrite (22 ml) and a solution of 3-fluoro-4-methylaniline (20.0g) in chloroform were added simultaneously. The mixture was then stirred at room temperature for 2 hours. The mixture was washed with water, hydrochloric acid (2 M), water, dried (MgSO₄) and filtered. The filtrate was evaporated to dryness to give 2-fluoro-4-(methylsulphenyl)toluene (25.37g) as a red oil which was not further purified.

By proceeding in a similar manner the following compound was prepared from the appropriately substituted starting material:
2-bromo-5-(ethylsulphenyl)benzotrifluoride, b.p. 105-112°C at 7mmHg.

### Example 5(a)

Hydrogen peroxide (30%, 62.3ml) was added dropwise to a stirred, cooled solution of 4-(ethylsulphenyl)-2-trifluoromethylbenzoic acid (20g) and acetic anhydride (10.1 ml) in acetic acid whilst maintaining the temperature below 10°C. The mixture was stirred at 0°C for 1 hour, room temperature for 2 hours and heated at 70°C for 2 hours. After cooling the mixture was poured onto ice and extracted with ethyl acetate. The organic extract was washed with water, aqueous ferrous sulphate solution, water, then dried (MgSO₄) and filtered. The filtrate was evaporated to dryness to give 4-(ethylsulphonyl)-2-trifluoromethylbenzoic acid (21.5g) as an off-white solid, NMR (CDCl₃) 1.25(t,3H) 3.15(q,2H) 7.8(d,1H) 8.0(d,1H) 8.1(s,1H).

### Example 6(a)

A solution of n-butyllithium in hexane (2.5m, 115 ml) was added dropwise with cooling to a stirred solution of 2-bromo-5-(ethylsulphenyl)benzotrifluoride (80.0 g) in anhydrous ether whilst maintaining the temperature below -70°C. The mixture was stirred at -78°C for 1.5 hours and poured onto solid carbon dioxide pallets. The mixture was stirred for 20 minutes then treated with hydrochloric acid (2 m). The layers were separated and the organic layer was washed with water, dried (MgSO₄) and filtered. The filtrate was evaporated to dryness. The residue was triturated with cyclohexane and filtered to give 4-(ethylsulphenyl)-2-trifluoromethylbenzoic acid as an off-white solid, m.p. 133.5°C.

According to a feature of the present invention, there is provided a method for controlling the growth of weeds (i.e. undesired vegetation) at a locus which comprises applying to the locus a herbicidally effective amount of at least one 2-cyano-1,3-dione derivative of general formula (I) or an agriculturally acceptable salt thereof. For this purpose, the 2-cyano-1,3-dione derivatives are normally used in the form of herbicidal compositions (i.e. in association with compatible diluents or carriers and/or surface active agents suitable for use in herbicidal compositions), for example as hereinafter described.

The compounds of general formula (I) show herbicidal activity against dicotyledonous (i.e. broad-leafed) and monocotyledonous (e.g. grass) weeds by pre- and/or post-emergence application.

By the term "pre-emergence application" is meant application to the soil in which the weed seeds or seedlings are present before emergence of the weeds above the surface of the soil. By the term "post-emergence application" is meant application to the aerial or exposed portions of the weeds which have emerged above the surface of the soil. For example, the compounds of general formula (I) may be used to control the growth of:
broad-leafed weeds, for example, Abutilon theophrasti, Amaranthus retroflexus, Bidens pilosa, Chenopodium album, Galium aparine, Ipomoea spp. e.g. Ipomoea purpurea, Sesbania exaltata, Sinapis arvensis, Solanum nigrum and Xanthium strumarium, and
grass weeds, for example Alopecurus myosuroides, Avena fatua, Digitaria sanguinalis, Echinochloa crus-galli, Eleusine indica and Setaria spp, e.g. Setaria faberii or Setaria viridis, and
sedges, for example, Cyperus esculentus.

The amounts of compounds of general formula (I) applied vary with the nature of the weeds, the compositions used, the time of application, the climatic and edaphic conditions and (when used to control the growth of weeds in crop-growing areas) the nature of the crops. When applied to a crop-growing area, the rate of application should be sufficient to control the growth of weeds without causing substantial permanent damage to the crop. In general, taking these factors into account, application rates between 0.01kg and 5kg of active material per hectare give good results. However, it is to be understood that higher or lower application rates may be used, depending upon the particular problem of weed control encountered.

The compounds of general formula (I) may be used to control selectively the growth of weeds, for example to control the growth of those species hereinbefore mentioned, by pre- or post-emergence application in a directional or non-directional fashion, e.g. by directional or non-directional spraying, to a locus of weed infestation which is an area used, or to be used, for growing crops, for example cereals, e.g. wheat, barley, oats, maize and rice, soya beans, field and dwarf beans, peas, lucerne, cotton, peanuts, flax, onions, carrots, cabbage, oilseed rape, sunflower, sugar beet, and permanent or sown grassland before or after sowing of the crop or before or after emergence of the crop. For the selective control of weeds at a locus of weed infestation which is an area used, or to be used, for growing of crops, e.g. the crops hereinbefore mentioned, application rates between 0.01kg and 4.0kg, and preferably between 0.01kg and 2.0kg, of active material per hectare are particularly suitable.

The compounds of general formula (I) may also be used to control the growth of weeds, especially those indicated above, by pre- or post-emergence application in established orchards and other tree-growing areas, for example forests, woods and parks, and plantations, e.g. sugar cane, oil palm and rubber plantations. For this purpose they may be applied in a directional or non- directional fashion (e.g. by directional or non-directional spraying) to the weeds or to the soil in which they are expected to appear, before or after planting of the trees or plantations at application rates between 0.25kg and 5.0kg, and preferably between 0.5kg and 4.0kg of active material per hectare.

The compounds of general formula (I) may also be used to control the growth of weeds, especially those indicated above, at loci which are not crop-growing areas but in which the control of weeds is nevertheless desirable.

Examples of such non-crop-growing areas include airfields, industrial sites, railways, roadside verges, the verges of rivers, irrigation and other waterways, scrublands and fallow or uncultivated land, in particular where it is desired to control the growth of weeds in order to reduce fire risks. When used for such purposes in which a total herbicidal effect is frequently desired, the active compounds are normally applied at dosage rates higher than those used in crop-growing areas as hereinbefore described. The precise dosage will depend upon the nature of the vegetation treated and the effect sought.

Pre- or post-emergence application, and preferably pre-emergence application, in a directional or non-directional fashion (e.g. by directional or non-directional spraying) at application rates between 1.0kg and 20.0kg, and preferably between 5.0 and 10.0kg, of active material per hectare are particularly suitable for this purpose.

When used to control the growth of weeds by pre-emergence application, the compounds of general formula (I) may be incorporated into the soil in which the weeds are expected to emerge. It will be appreciated that when the compounds of general formula (I) are used to control the growth of weeds by post-emergence application, i.e. by application to the aerial or exposed portions of emerged weeds, the compounds of general formula (I) will also normally come into contact with the soil and may also then exercise a pre-emergence control on later-germinating weeds in the soil.

Where especially prolonged weed control is required, the application of the compounds of general formula (I) may be repeated if required.

According to a further feature of the present invention, there are provided compositions suitable for herbicidal use comprising one or more of the 2-cyano-1,3-dione derivatives of general formula (I) or an agriculturally acceptable salt thereof in association with, and preferably homogeneously dispersed in, one or more compatible herbicidally- acceptable diluents or carriers and/or surface active agents [i.e. diluents or carriers and/or surface active agents of the type generally accepted in the art as being suitable for use in herbicidal compositions and which are compatible with compounds of general formula (I)]. The term "homogeneously dispersed" is used to include compositions in which the compounds of general formula (I) are dissolved in other components. The term "herbicidal compositions" is used in a broad sense to include not only compositions which are ready for use as herbicides but also concentrates which must be diluted before use. Preferably, the compositions contain from 0.05 to 90% by weight of one or more compounds of general formula (I).

The herbicidal compositions may contain both a diluent or carrier and surface-active (e.g. wetting, dispersing, or emulsifying) agent. Surface-active agents which may be present in herbicidal compositions of the present invention may be of the ionic or non-ionic types, for example sulphoricinoleates, quaternary ammonium derivatives, products based on condensates of ethylene oxide with alkyl and polyaryl phenols, e.g. nonyl- or octyl-phenols, or carboxylic acid esters of anhydrosorbitols which have been rendered soluble by etherification of the free hydroxy groups by condensation with ethylene oxide, alkali and alkaline earth metal salts of sulphuric acid esters and sulphonic acids such as dinonyl- and dioctyl-sodium sulphonosuccinates and alkali and alkaline earth metal salts of high molecular weight sulphonic acid derivatives such as sodium and calcium lignosulphonates and sodium and calcium alkylbenzene sulphonates.

Suitably, the herbicidal compositions according to the present invention may comprise up to 10% by weight, e.g. from 0.05% to 10% by weight, of surface-active agent but, if desired, herbicidal compositions according to the present invention may comprise higher proportions of surface-active agent, for example up to 15% by weight in liquid emulsifiable suspension concentrates and up to 25% by weight in liquid water soluble concentrates. Examples of suitable solid diluents or carriers are aluminium silicate, talc, calcined magnesia, kieselguhr, tricalcium phosphate, powdered cork, adsorbent carbon black and clays such as kaolin and bentonite. The solid compositions (which may take the form of dusts, granules or wettable powders) are preferably prepared by grinding the compounds of general formula (I) with solid diluents or by impregnating the solid diluents or carriers with solutions of the compounds of general formula (I) in volatile solvents, evaporating the solvents and, if necessary, grinding the products so as to obtain powders. Granular formulations may be prepared by absorbing the compounds of general formula (I) (dissolved in suitable solvents, which may, if desired, be volatile) onto the solid diluents or carriers in granular form and, if desired, evaporating the solvents, or by granulating compositions in powder form obtained as described above. Solid herbicidal compositions, particularly wettable powders and granules, may contain wetting or dispersing agents (for example of the types described above), which may also, when solid, serve as diluents or carriers.

Liquid compositions according to the invention may take the form of aqueous, organic or aqueous-organic solutions, suspensions and emulsions which may incorporate a surface-active agent. Suitable liquid diluents for incorporation in the liquid compositions include water, glycols, tetrahydrofurfuryl alcohol, acetophenone, cyclohexanone, isophorone, toluene, xylene, mineral, animal and vegetable oils and light aromatic and naphthenic fractions of petroleum (and mixtures of these diluents). Surface-active agents, which may be present in the liquid compositions, may be ionic or non-ionic (for example of the types described above) and may, when liquid, also serve as diluents or carriers.

Powders, dispersible granules and liquid compositions in the form of concentrates may be diluted with water or other suitable diluents, for example mineral or vegetable oils, particularly in the case of liquid concentrates in which the diluent or carrier is an oil, to give compositions ready for use.

When desired, liquid compositions of the compound of general formula (I) may be used in the form of self-emulsifying concentrates containing the active substances dissolved in the emulsifying agents or in solvents containing emulsifying agents compatible with the active substances, the simple addition of water to such concentrates producing compositions ready for use.

Liquid concentrates in which the diluent or carrier is an oil may be used without further dilution using the electrostatic spray technique.

Herbicidal compositions according to the present invention may also contain, if desired, conventional adjuvants such as adhesives, protective colloids, thickeners, penetrating agents, stabilisers, sequestering agents, anti-caking agents, colouring agents and corrosion inhibitors. These adjuvants may also serve as carriers or diluents. Unless otherwise specified, the following percentages are by weight. Preferred herbicidal compositions according to the present invention are
aqueous suspension concentrates which comprise from 10 to 70% of one or more compounds of general formula (I), from 2 to 10% of surface-active agent, from 0.1 to 5% of thickener and from 15 to 87.9% of water;
wettable powders which comprise from 10 to 90% of one or more compounds of general formula (I), from 2 to 10% of surface-active agent and from 8 to 88% of solid diluent or carrier;
soluble powders which comprise from 10 to 90% of one or more compounds of general formula (I), from 2 to 40% of sodium carbonate and from 0 to 88% of solid diluent;
liquid water soluble concentrates which comprise from 5 to 50%, e.g. 10 to 30%, of one or more compounds of general formula (I), from 5 to 25% of surface-active agent and from 25 to 90%, e.g. 45 to 85%, of water miscible solvent, e.g. dimethylformamide, or a mixture of water-miscible solvent and water;
liquid emulsifiable suspension concentrates which comprise from 10 to 70% of one or more compounds of general formula (I), from 5 to 15% of surface-active agent, from 0.1 to 5% of thickener and from 10 to 84.9% of organic solvent;
granules which comprise from 1 to 90%, e.g. 2 to 10% of one or more compounds of general formula (I), from 0.5 to 7%, e.g. 0.5 to 2%, of surface-active agent and from 3 to 98.5%, e.g. 88 to 97.5%, of granular carrier and
emulsifiable concentrates which comprise 0.05 to 90%, and preferably from 1 to 60% of one or more compounds of general formula (I), from 0.01 to 10%, and preferably from 1 to 10%, of surface-active agent and from 9.99 to 99.94%, and preferably from 39 to 98.99%, of organic solvent.

Herbicidal compositions according to the present invention may also comprise the compounds of general formula (I) in association with, and preferably homogeneously dispersed in, one or more other pesticidally active compounds and, if desired, one or more compatible pesticidally acceptable diluents or carriers, surface-active agents and conventional adjuvants as hereinbefore described. Examples of other pesticidally active compounds which may be included in, or used in conjunction with, the herbicidal compositions of the present invention include herbicides, for example to increase the range of weed species controlled for example alachlor [2-chloro-2,6'-diethyl-N-(methoxymethyl)-acetanilide], atrazine [2-chloro-4-ethylamino-6-isopropylamino-1,3,5-triazine], bromoxynil [3,5-dibromo-4-hydroxybenzonitrile], chlortoluron [N'-(3-chloro-4-methylphenyl)-N,N-dimethylurea], cyanazine [2-chloro-4-(1-cyano-1-methylethylamino)-6-ethylamino-1,3,5-triazine], 2,4-D [2,4-dichlorophenoxyacetic acid], dicamba [3,6-dichloro-2-methoxybenzoic acid], difenzoquat [1,2-dimethyl-3,5-diphenyl-pyrazolium salts], flampropmethyl [methyl N-2-(N-benzoyl-3-chloro-4-fluoroanilino)-propionate], fluometuron [N'-(3-trifluoromethylphenyl)-N,N-dimethylurea], isoproturon [N'-(4-isopropylphenyl)-N,N-dimethylurea], nicosulfuron [2-(4,6-dimethoxypyrimidin-2-ylcarbamoylsulfamoyl)-N,N-dimethylnicotinamide], insecticides, e.g. synthetic pyrethroids, e.g. permethrin and cypermethrin, and fungicides, e.g. carbamates, e.g. methyl N-(1-butyl-carbamoyl- benzimidazol-2-yl)carbamate, and triazoles e.g. 1-(4-chlorophenoxy)-3,3- dimethyl-1-(1,2,4-triazol-1-yl)-butan-2-one.

Pesticidally active compounds and other biologically active materials which may be included in, or used in conjunction with, the herbicidal compositions of the present invention, for example those hereinbefore mentioned, and which are acids, may, if desired, be utilized in the form of conventional derivatives, for example alkali metal and amine salts and esters.

According to a further feature of the present invention there is provided an article of manufacture comprising at least one of the 2-cyano-1,3-dione derivatives of general formula (I) or an agriculturally acceptable salt thereof or, as is preferred, a herbicidal composition as hereinbefore described, and preferably a herbicidal concentrate which must be diluted before use, comprising at least one of the 2-cyano-1,3-dione derivatives of general formula (I) within a container for the aforesaid derivative or derivatives of general formula (I), or a said herbicidal composition, and instructions physically associated with the aforesaid container setting out the manner in which the aforesaid derivative or derivatives of general formula (I) or herbicidal composition contained therein is to be used to control the growth of weeds. The containers will normally be of the types conventionally used for the storage of chemical substances which are solid at normal ambient temperatures and herbicidal compositions particularly in the form of concentrates, for example cans and drums of metal, which may be internally lacquered, and plastics materials, bottles or glass and plastics materials and, when the contents of the container is a solid, for example granular, herbicidal compositions, boxes, for example of cardboard, plastics materials and metal, or sacks. The containers will normally be of sufficient capacity to contain amounts of the 2-cyano-1,3-dione derivative or herbicidal compositions sufficient to treat at least one acre of ground to control the growth of weeds therein but will not exceed a size which is convenient for conventional methods of handling. The instructions will be physically associated with the container, for example by being printed directly thereon or on a label or tag affixed thereto. The directions will normally indicate that the contents of the container, after dilution if necessary, are to be applied to control the growth of weeds at rates of application between 0.01kg and 20kg of active material per hectare in the manner and for the purposes hereinbefore described.

The following Examples illustrate herbicidal compositions according to the present invention:

### EXAMPLE C1

A wettable powder was formed from:
* active ingredient (compound A): 50% w/w
* nonylphenol/ethylene oxide condensate containing 9 moles of ethylene oxide per mol of phenol: 5% w/w
* silicon dioxide of micro-fine particle size: 5% w/w
* synthetic magnesium silicate carrier: 40% w/w
by absorbing the condensate on the silicon dioxide, mixing with the other ingredients and grinding the mixture in a hammermill to give a wettable powder.

Similar wettable powders may be prepared as described above by replacing the 2-cyano-1,3-dione (compound A) by other compounds of general formula (I).

### EXAMPLE C2

An aqueous suspension concentrate was formed from:
* active ingredient (compound A): 50% w/v
* nonylphenol/ethylene oxide condensate containing 9 moles of ethylene oxide per mol of phenol: 1 % w/v
* sodium salt of polycarboxylic acid: 0.2% w/v
* Ethylene glycol: 5% w/v
* polysaccaride xanthan gum thickener: 0.15% w/v
* water to 100% by volume
by intimately mixing the ingredients and grinding in a ball-mill for 24 hours.

Similar aqueous concentrates may be prepared as described above by replacing the 2-cyano-1,3-dione (compound A) by other compounds of general formula (I).

Representative compounds of general formula (I) have been used in herbicidal applications according to the following procedures.

### Method of use of herbicidal compounds:

### Herbicidal activity:

Appropriate quantities of the compounds used to treat the plant were dissolved in acetone to give solutions equivalent to an application rate of up to 4000g of the compounds used to treat the plants per hectare (g/ha). These solutions were applied at 260 litres of spray fluid per hectare.

### a) Pre-emergence application weed control.

Seeds (weeds or crops) were sown in loam soil pots.

The compounds of the invention were applied to the soil surface as described above.

### b) Post-emergence application weed control.

Weed species were grown until ready for spraying with the compounds of the invention . The growth stage of the plants at spraying were as follows :

| 1) Broad-leafed weeds : | |
|---|---|
| Abutilon theophrasti : | 1-2 leaves. |
| Amaranthus retroflexus: | 1-2 leaves. |
| Galium aparine: | 1-2 whorls. |
| Sinapis arvensis : | 2 leaves. |
| Ipomoea purpurea : | 1-2 leaves. |
| Xanthium strumarium: | 2 leaves. |

| 2) Grass weeds : | |
|---|---|
| Alopecurus myosuroides: | 2 leaves. |
| Avena fatua : | 1-2 leaves. |
| Echinochloa crus-galli | 2-3 leaves. |
| Setaria viridis : | 2-3 leaves. |

| 3) Sedges : | |
|---|---|
| Cyperus esculentus : | 3 leaves. |

### c) Crop tolerance

Compounds of the invention were applied pre-emergence as in (a) or post emergence (3-leaf stage) to the following crops :- wheat, maize, rice, soya and cotton.

A single pot of each plant species was allocated to each treatment with unsprayed controls and controls sprayed with acetone alone.

After treatment, the pots were kept in the greenhouse and were watered overhead.

Visual assessment of phytotoxicity was made 17-20 days after spraying. Weed control results were expressed as the percentage reduction in growth or kill of the weeds, in comparison with the plants in the control pots. Crop tolerance was expressed as the percentage damage to crop.

Representative compounds of the invention, when used at an application rate of 4kg/ha or less, show herbicidal activity against one or more of the weed species listed above: such compounds also show selectivity on one or more of the listed crop species.

## Claims

1. A 2-cyano-1,3-dione derivative of general formula I: 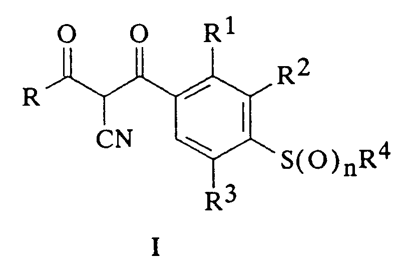 wherein:
R represents:-
a straight- or branched-chain alkyl group containing up to 6 carbon atoms which is optionally substituted by one or more halogen atoms which may be the same or different; or
a cycloalkyl group containing from 3 to 6 carbon atoms which is optionally substituted by one or more groups selected from R⁵ and one or more halogen atoms which may be the same or different;
R¹ represents:-
a halogen atom, or
a straight- or branched-chain alkyl group containing up to 6 carbon atoms which is substituted by -OR⁵; or
a group selected from R⁵, SR⁵, cyano, -OR⁵, -O(CH₂)ₘOR⁵, or -CO₂R⁵;
R² and R³, which may be the same or different, each represents:-
a halogen or hydrogen atom, or
a straight- or branched-chain alkyl group containing up to 6 carbon atoms which is substituted by -OR⁵; or
a group selected from R⁵, nitro, cyano, -OR⁵, -O(CH₂)ₘOR⁵ or -CO₂R⁵;
provided that at least one of R² and R³ is hydrogen;
R⁴ and R⁵, which may be the same or different, each represents:-
a straight- or branched-chain alkyl group containing up to 6 carbon atoms which is optionally substituted by one or more halogen atoms which may be the same or different;
n is zero, 1 or 2; m is an integer from 1 to 3;
an enolic tautomeric form thereof,
or an agriculturally acceptable salt of the enolic tautomeric form.

2. A compound according to claim 1 wherein:
(a) R represents ethyl, methyl, n-propyl, isopropyl, t-butyl, cyclopropyl or 1-methylcyclopropyl; and/or
(b) R¹ represents:-
a halogen atom; or
a group selected from -OR⁵ and R⁵; and/or
(c) R² and R³, which may be the same or different, each represents:-
a halogen or hydrogen atom; or
a straight- or branched-chain alkyl group containing up to 6 carbon atoms which is substituted by -OR⁵ or
a group selected from R⁵, -OR⁵,
-O(CH₂)ₘOR⁵ where m is 2 or 3,
or -CO₂R⁵,
provided that at least one of R² and R³ is hydrogen; and/or
(d) R⁴ represents:-
a straight- or branched-chain alkyl group containing up to 4 carbon atoms which is optionally substituted by one or more halogen atoms which may be the same or different; and/or
(e) R⁵ represents:-
a straight- or branched-chain alkyl group containing up to 4 carbon atoms which is optionally substituted by one or more halogen atoms which may be the same or different; and
(f) 'halogen' means chlorine, bromine or fluorine.

3. A compound according to claim 1 wherein R³ represents hydrogen.

4. A compound according to claim 1 wherein:
R¹ represents a group selected from R⁵, cyano, -SR⁵, -O(CH₂)ₘOR⁵ and -CO₂R⁵; and
R⁵ represents a straight- or branched-chain alkyl group containing up to 6 carbon atoms.

5. A compound according to claim 1 wherein:
R represents methyl, ethyl, isopropyl, t-butyl, cyclopropyl or 1-methylcyclopropyl;
R¹ represents chlorine, bromine, fluorine, trifluoromethyl or methoxy;
R² represents hydrogen, chlorine or methoxy;
R³ represents hydrogen;
R⁴ represents methyl or ethyl; and
n is 0, 1 or 2.

6. A compound according to claim 1 which is
1-[2-chloro-4-(methylsulphonyl)phenyl]-2-cyano-4,4-dimethylpentan-1,3-dione,
1-[2-chloro-4-(methylsulphonyl)phenyl]-2-cyano-3-cyclopropylpropan-1,3-dione,
2-cyano-3-cyclopropyl-1-[4-(methylsulphonyl)-2-trifluoromethylphenyl]propan-1,3-dione,
1-[2-chloro-4-(methylsulphonyl)phenyl]-2-cyanopentan-1,3-dione,
1-[2-chloro-4-(methylsulphonyl)phenyl]-2-cyano-4-methylhexan-1,3-dione,
1-[2-chloro-4-(methylsulphonyl)phenyl]-2-cyanohexan-1,3-dione,
2-cyano-4,4-dimethyl-1-[4-(methylsulphonyl)-2-trifluoromethylphenyl]pentan-1,3-dione,
2-cyano-1-[4-(methylsulphonyl)-2-trifluoromethylphenyl]-3-(1-methylcyclopropyl)propan-1,3-dione,
2-cyano-1-[4-(methylsulphonyl)-2-trifluoromethylphenyl]butan-1,3-dione,
1-(2-chloro-4-(methylsulphenyl)phenyl)-2-cyano-3-cyclopropylpropan-1,3-dione,
2-cyano-3-cyclopropyl-1-(2-fluoro-4-(methylsulphonyl)phenyl) propan-1,3-dione,
1-[2-chloro-4-(ethylsulphonyl)phenyl]-2-cyano-3-cyclopropylpropan-1,3-dione,
2-cyano-3-cyclopropyl-1-[4-(methylsulphenyl)-2-trifluoromethylphenyl]propan-1,3-dione,
2-cyano-3-cyclopropyl-1-[2-(methoxy-4-(methylsulphenyl)phenyl]propan-1,3-dione,
1-[2-bromo-3-methoxy-4-(methylsulphonyl)phenyl]-2-cyanobutan-1,3-dione,
1-[2,3-dichloro-4-(methylsulphonyl)-phenyl]-2-cyano-3-(1-methylcyclopropyl)propan-1,3-dione,
1-[2-chloro-3-methoxy-4-(methylsulphonyl)phenyl]-2-cyano-3-cyclopropylpropan-1,3-dione,
1-[2-chloro-3-methoxy-4-(methylsulphonyl)phenyl]-2-cyanobutan-1,3-dione,
1-[2-bromo-4-(methylsulphonyl)phenyl]-2-cyano-3-cyclopropylpropan-1,3-dione,
1-[2-bromo-4-(methylsulphonyl)phenyl]-2-cyano-3-(1-methylcyclopropyl)propan 1,3-dione,
2-cyano-3-(1-methylcyclopropyl)-1-[4-(ethylsulphonyl)-2-trifluoromethylphenyl]propan-1,3-dione, or
1-[2-chloro-3-methoxy-4-(methylsulphonyl)phenyl]-2-cyano-3-(1-methylcyclopropyl)propan-1,3-dione,
or an agriculturally acceptable salt of an enolic tautomeric form thereof.

7. A process for the preparation of a compound according to claim 1 which comprises:
a) the reaction of a beta-ketonitrile of formula II with a benzoyl chloride of formula III in which n is 0 or 2: 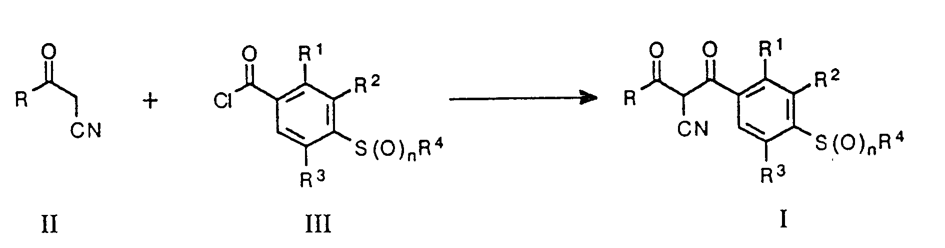 wherein R, R¹, R², R³ and R⁴ are as defined in claim 1 and n is 0 or 2;
b) the reaction of an acid chloride of formula IV, with a beta-ketonitrile of formula V in which n is 0 or 2: 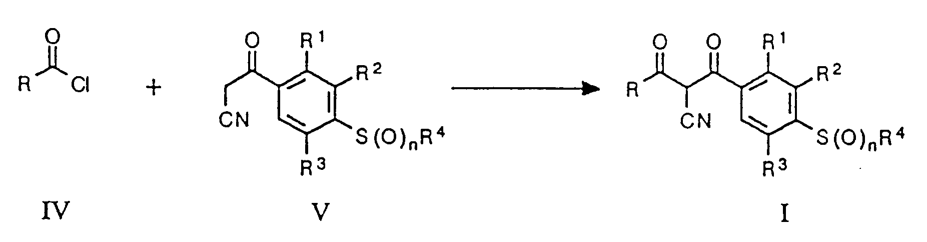 wherein R, R¹, R², R³ and R⁴ are as defined in claim 1; or
c) optionally oxidising a compound of formula I wherein n is 0 to a compound wherein n is 1 or 2;
and optionally converting a 2-cyano-1,3-dione of general formula I thus obtained into an agriculturally acceptable salt thereof.

8. A process according to claim 7 wherein the reaction is carried out in the presence of a base in a solvent or solvent mixture at a temperature from 0°C to the boiling point of the solvent.

9. A herbicidal composition which comprises as an active ingredient a herbicidally effective amount of a 2-cyano-1,3-dione derivative of general formula I according to any one of claims 1 to 6 or an agriculturally acceptable salt thereof and a herbicidally acceptable diluent or carrier, and optionally one or more surface active agents.

10. A herbicidal composition according to claim 9 which comprises from 0.05 to 90% by weight of active ingredient.

11. A herbicidal composition according to claim 9 or 10 which comprises from 0.05% to 25% by weight of surface active agent, and optionally an adhesive, protective colloid, thickener, penetrating agent, stabiliser, sequestering agent, anti-caking agent, colouring agent and/or corrosion inhibitor.

12. A herbicidal composition according to claim 9, 10 or 11 which is in the form of
an aqueous suspension concentrate, a wettable powder, a soluble powder, a liquid water soluble concentrate, a liquid emulsifiable suspension concentrate,a granule or an emulsifiable concentrate.

13. A method of controlling the growth of weeds at a locus which comprises applying thereto a 2-cyano-1,3-dione derivative of general formula I according to any one of claims 1 to 6 or an agriculturally acceptable salt thereof.

14. A method according to claim 13 wherein the locus is an area used, or to be used, for growing crops, using an application rate of between 0.01 kg and 4.0 kg of active material per hectare.

15. A method according to claim 13 wherein the locus is not a crop-growing area, using an application rate of between 1.0 kg and 20.0 kg of active material per hectare.

## Patentansprüche

1. 2-Cyano-1,3-dionderivat der allgemeinen Formel I: 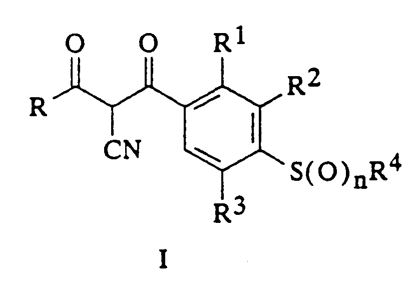 wobei
R eine gerad- oder verzweigtkettige Alkylgruppe mit bis zu 6 Kohlenstoffatomen, die gegebenenfalls durch ein oder mehrere Halogenatome substituiert ist, die gleich oder verschieden sein können, oder
eine Cycloalkylgruppe mit 3 bis 6 Kohlenstoffatomen, die gegebenenfalls substituiert ist kann durch eine oder mehrere Gruppen, ausgewählt aus R⁵ und einem oder mehreren Halogenatomen, die gleich oder verschieden sein können, bedeutet;
R¹ ein Halogenatom oder
eine gerad- oder verzweigtkettige Alkylgruppe mit bis zu 6 Kohlenstoffatomen, die substituiert ist durch -OR⁵, oder
eine Gruppe, ausgewählt aus R⁵, SR⁵, Cyano, -OR⁵, -O(CH₂)ₘOR⁵ oder -CO₂R⁵, bedeutet;
R² und R³, die gleich oder verschieden sein können, jeweils ein Halogen- oder Wasserstoffatom, oder
eine gerad- oder verzweigtkettige Alkylgruppe mit bis zu 6 Kohlenstoffatomen, die durch -OR⁵ substituiert ist, oder
eine Gruppe, ausgewählt aus R⁵, Nitro, Cyano, -OR⁵, -O(CH₂)ₘOR⁵ oder -CO₂R⁵, bedeuten,
mit der Maßgabe, daß mindestens einer der Reste R² und R³ Wasserstoff ist;
R⁴ und R⁵, die gleich oder verschieden sein können, jeweils eine gerad- oder verzweigtkettige Alkylgruppe mit bis zu 6 Kohlenstoffatomen, die gegebenenfalls durch ein oder mehrere Halogenatome, die gleich oder verschieden sein können, substituiert ist, bedeuten;
n 0, 1 oder 2 ist;
m eine ganze Zahl von 1 bis 3 ist;
eine enolische tautomere Form davon,
oder ein landwirtschaftlich annehmbares Salz der enolischen tautomeren Form.

2. Verbindung nach Anspruch 1, wobei
(a) R Ethyl, Methyl, n-Propyl, Isopropyl, t-Butyl, Cyclopropyl oder 1-Methylcyclopropyl bedeutet und/oder
(b) R¹
ein Halogenatom oder
eine Gruppe, ausgewählt aus -OR⁵ und R⁵, bedeutet und/oder
(c) R² und R³, die gleich oder verschieden sein können, jeweils ein Halogen- oder Wasserstoffatom oder
eine gerad- oder verzweigtkettige Alkylgruppe mit bis zu 6 Kohlenstoffatomen, die durch -OR⁵ substituiert ist, oder
eine Gruppe, ausgewählt aus R⁵, -OR⁵, -O(CH₂)ₘOR⁵, wobei m 2 oder 3 ist, oder
-CO₂R⁵ bedeuten,
mit der Maßgabe, daß mindestens einer der Reste R² und R³ Wasserstoff ist, und/oder
(d) R⁴ eine gerad- oder verzweigtkettige Alkylgruppe, enthaltend bis zu 4 Kohlenstoffatome, die gegebenenfalls durch ein oder mehrere Halogenatome, die gleich oder verschieden sein können, substituiert ist, bedeutet und/oder
(e) R⁵ eine gerad- oder verzweigtkettige Alkylgruppe mit bis zu 4 Kohlenstoffatomen, die gegebenenfalls durch ein oder mehrere Halogenatome substituiert ist, die gleich oder verschieden sein können, bedeutet und
(f) "Halogen" Chlor, Brom oder Fluor bedeutet.

3. Verbindung nach Anspruch 1, wobei R³ Wasserstoff bedeutet.

4. Verbindung nach Anspruch 1, wobei
R¹ eine Gruppe bedeutet, ausgewählt aus R⁵, Cyano, -SR⁵, -O(CH₂)ₘOR⁵ und -CO₂R⁵, und
R⁵ eine gerad- oder verzweigtkettige Alkylgruppe mit bis zu 6 Kohlenstoffatomen bedeutet.

5. Verbindung nach Anspruch 1, wobei
R Methyl, Ethyl, Isopropyl, t-Butyl, Cyclopropyl oder 1-Methylcyclopropyl bedeutet,
R¹ Chlor, Brom, Fluor, Trifluormethyl oder Methoxy bedeutet,
R² Wasserstoff, Chlor oder Methoxy bedeutet,
R³ Wasserstoff bedeutet,
R⁴ Methyl oder Ethyl bedeutet und
n 0, 1 oder 2 ist.

6. Verbindung nach Anspruch 1, die
1-[2-Chlor-4-(methylsulfonyl)phenyl]-2-cyano-4,4-dimethylpentan-1,3-dion,
1-[2-Chlor-4-(methylsulfonyl)phenyl]-2-cyano-3-cyclopropylpropan-1,3-dion,
2-Cyano-3-cyclopropyl-1-[4-(methylsulfonyl)-2-trifluormethylphenyl]propan-1,3-dion,
1-[2-Chlor-4-(methylsulfonyl)phenyl]-2-cyanopentan-1,3-dion,
1-[2-Chlor-4-(methylsulfonyl)phenyl]-2-cyano-4-methylhexan-1,3-dion,
1-[2-Chlor-4-(methylsulfonyl)phenyl]-2-cyanohexan-1,3-dion,
2-Cyano-4,4-dimethyl-1-[4-(methylsulfonyl)-2-trifluormethylphenyl]pentan-1,3-dion,
2-Cyano-1-[4-(methylsulfonyl)-2-trifluormethylphenyl]-3-(1-methylcyclopropyl)propan-1,3-dion,
2-Cyano-1-[4-(methylsulfonyl)-2-trifluormethylphenyl]butan-1,3-dion,
1-(2-Chlor-4-(methylsulfenyl)phenyl)-2-cyano-3-cyclopropylpropan-1,3-dion,
2-Cyano-3-cyclopropyl-1-(2-fluor-4-(methylsulfonyl)phenyl)propan-1,3-dion,
1-[2-Chlor-4-(ethylsulfonyl)phenyl]-2-cyano-3-cyclopropylpropan-1,3-dion,
2-Cyano-3-cyclopropyl-1-[4-(methylsulfenyl)-2-trifluormethylphenyl]-propan-1,3-dion,
2-Cyano-3-cyclopropyl-1-[2-methoxy-4-(methylsulfenyl)phenyl]propan-1,3-dion,
1-[2-Brom-3-methoxy-4-(methylsulfonyl)phenyl]-2-cyanobutan-1,3-dion,
1-[2,3-Dichlor-4-(methylsulfonyl)phenyl]-2-cyano-3-(1-methylcyclopropyl)propan-1,3-dion,
1-[2-Chlor-3-methoxy-4-(methylsulfonyl)phenyl]-2-cyano-3-cyclopropylpropan-1,3-dion,
1-[2-Chlor-3-methoxy-4-(methylsulfonyl)phenyl]-2-cyanobutan-1,3-dion,
1-[2-Brom-4-(methylsulfonyl)phenyl]-2-cyano-3-cyclopropylpropan-1,3-dion,
1-[2-Brom-4-(methylsulfonyl)phenyl]-2-cyano-3-(1-methylcyclopropyl)propan-1,3-dion,
2-Cyano-3-(1-methylcyclopropyl)-1-[4-(ethylsulfonyl)-2-trifluormethylphenyl]propan-1,3-dion oder
1-[2-Chlor-3-methoxy-4-(methylsulfonyl)phenyl]-2-cyano-3-(1-methylcyclopropyl)propan-1,3-dion ist, oder
ein landwirtschaftlich annehmbares Salz oder eine enolische tautomere Form davon.

7. Verfahren zur Herstellung einer Verbindung nach Anspruch 1, umfassend
a) die Umsetzung eines β-Ketonitrils der Formel II mit einem Benzoylchlorid der Formel III, in der n 0 oder 2 ist 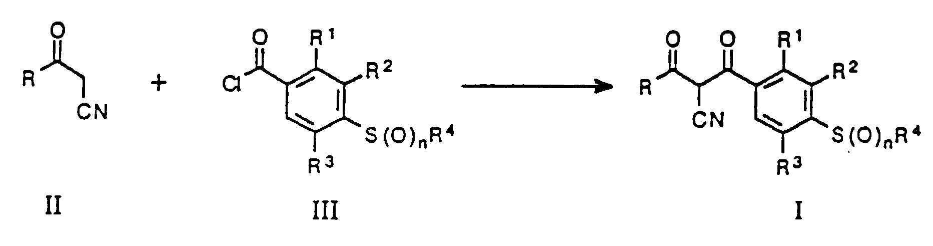 wobei R, R¹, R², R³ und R⁴ wie in Anspruch 1 definiert sind, und n 0 oder 2 ist;
b) Umsetzung eines Säurechlorids der Formel IV mit einem β-Ketonitril der Formel V, in der n 0 oder 2 ist 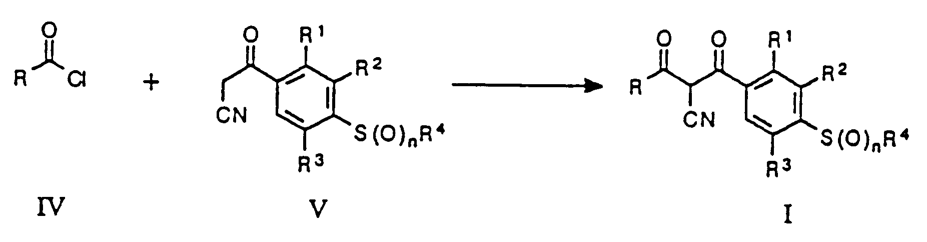 wobei R, R¹, R², R³ und R⁴ wie in Anspruch 1 definiert sind, oder
c) gegebenenfalls Oxidieren einer Verbindung der Formel I, in der n 0 ist, zu einer Verbindung, in der n 1 oder 2 ist, und
gegebenenfalls Umwandlung eines so erhaltenen 2-Cyano-1,3-dions der allgemeinen Formel I in ein landwirtschaftlich annehmbares Salz davon.

8. Verfahren nach Anspruch 7, wobei die Reaktion in Gegenwart einer Base in einem Lösungsmittel oder Lösungsmittelgemisch bei einer Temperatur von 0°C bis zum Siedepunkt des Lösungsmittels durchgeführt wird.

9. Herbizides Mittel, umfassend als Wirkstoff eine herbizid wirksame Menge eines 2-Cyano-1,3-dionderivats der allgemeinen Formel I nach einem der Ansprüche 1 bis 6 oder eines landwirtschaftlich annehmbaren Salzes davon und ein für Herbizide annehmbares Verdünnungsmittel oder einen Träger, und gegebenenfalls ein oder mehrere grenzflächenaktive Mittel.

10. Herbizides Mittel nach Anspruch 9, umfassend 0,05 bis 90 Gew.-% Wirkstoff.

11. Herbizides Mittel nach Anspruch 9 oder 10, umfassend 0,05 bis 25 Gew.-% grenzflächenaktives Mittel und gegebenenfalls ein Haftmittel, Schutzkolloid, Verdickungsmittel, Penetrationsmittel, einen Stabilisator, ein Maskierungsmittel, ein Antizusammenbackmittel, ein Farbmittel und/oder einen Korrosionshemmer.

12. Herbizides Mittel nach Anspruch 9, 10 oder 11 in Form eines wäßrigen Suspensionskonzentrats, eines netzbaren Pulvers, eines löslichen Pulvers, eines flüssigen wasserlöslichen Konzentrats, eines flüssigen emulgierbaren Suspensionskonzentrats, eines Granulats oder eines emulgierbaren Konzentrats.

13. Verfahren zur Bekämpfung des Wachstums von Unkräutern an einer Stelle, umfassend das Aufbringen eines 2-Cyano-1,3-dionderivats der allgemeinen Formel I, nach einem der Ansprüche 1 bis 6 oder eines landwirtschaftlich annehmbaren Salzes davon auf die Stelle.

14. Verfahren nach Anspruch 13, wobei die Stelle ein Bereich ist, der zum Wachstum von Nutzpflanzen verwendet wird oder verwendet werden soll, unter Anwendung einer Aufbringmenge zwischen 0,01 kg und 4,0 kg Wirkstoff pro Hektar.

15. Verfahren nach Anspruch 13, wobei die Stelle ein nicht von Nutzpflanzen bewachsener Bereich ist, unter Anwendung einer Aufbringmenge zwischen 1,0 kg und 20 kg Wirkstoff pro Hektar.

## Revendications

1. Dérivé de 2-cyano-1,3-dione de formule générale I : 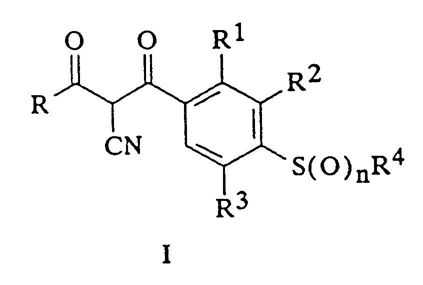 dans laquelle
R représente :
un groupe alkyle à chaîne droite ou à chaîne ramifiée contenant jusgu'à 6 atomes de carbone, qui est facultativement substitué par un ou plusieurs atomes d'halogènes qui peuvent être identiques ou différents ; ou bien
un groupe cycloalkyle contenant 3 à 6 atomes de carbone, qui est facultativement substitué par un ou plusieurs groupes choisis entre R⁵ et un ou plusieurs atomes d'halogènes qui peuvent être identiques ou différents ;
R¹ représente :
un atome d'halogène, ou
un groupe alkyle à chaîne droite ou à chaîne ramifiée contenant jusqu'à 6 atomes de carbone, qui est substitué par -OR⁵ ; ou bien
un groupe choisi entre R⁵, SR⁵, cyano, -OR⁵, -O(CH₂)ₘOR⁵ ou -CO₂R⁵ ;
R² et R³, qui peuvent être identiques ou différents, représentent chacun :
un atome d'halogène ou d'hydrogène, ou bien
un groupe alkyle à chaîne droite ou à chaîne ramifiée contenant jusqu'à 6 atomes de carbone, qui est substitué par -OR⁵ ; ou bien
un groupe choisi entre R⁵, nitro, cyano, -OR⁵, -O(CH₂)ₘOR⁵ ou -CO₂R⁵ ;
sous réserve que l'un au moins de R² et R³ soit de l'hydrogène ;
R⁴ et R⁵, qui peuvent être identiques ou différents, représentent chacun :
un groupe alkyle à chaîne droite ou à chaîne ramifiée contenant jusqu'à 6 atomes de carbone, qui est facultativement substitué par un ou plusieurs atomes d'halogènes qui peuvent être identiques ou différents ;
n a la valeur zéro, 1 ou 2 ; m est un nombre entier de 1 à 3 ;
une forme tautomérique énolique de ce dérivé,
ou un sel acceptable en agriculture de la forme tautomérique énolique.

2. Composé suivant la revendication 1, dans lequel :
(a) R représente un groupe éthyle, méthyle, n-propyle, isopropyle, tertio-butyle, cyclopropyle ou 1-méthylcyclopropyle ; et/ou
(b) R¹ représente :
un atome d'halogène ; ou
un groupe choisi entre -OR⁵ et R⁵ ; et/ou
(c) R² et R³, qui peuvent être identiques ou différents, représentent chacun :
un atome d'halogène ou d'hydrogène ; ou bien
un groupe alkyle à chaîne droite ou à chaîne ramifiée contenant jusqu'à 6 atomes de carbone, qui est substitué par -OR⁵, ou bien
un groupe choisi entre R⁵, -OR⁵,
-O(CH₂)ₘOR⁵, où m a la valeur 2 ou 3,
ou -CO₂R⁵,
sous réserve que l'un au moins de R² et R³ soit de l'hydrogène ; et/ou
(d) R⁴ représente :
un groupe alkyle à chaîne droite ou à chaîne ramifiée contenant jusqu'à 4 atomes de carbone, qui est facultativement substitué par un ou plusieurs atomes d'halogènes qui peuvent être identiques ou différents ; et/ou
(e) R⁵ représente :
un groupe alkyle à chaîne droite ou à chaîne ramifiée contenant jusqu'à 4 atomes de carbone, qui est facultativement substitué par un ou plusieurs atomes d'halogènes qui peuvent être identiques ou différents ; et
(f) le terme "halogène" désigne le chlore, le brome ou le fluor.

3. Composé suivant la revendication 1, dans lequel R³ représente l'hydrogène.

4. Composé suivant la revendication 1, dans lequel :
R¹ représente un groupe choisi entre R⁵, cyano, -SR⁵, -O(CH₂)ₘOR⁵ et -CO₂R⁵ ; et
R⁵ représente un groupe alkyle à chaîne droite ou à chaîne ramifiée contenant jusqu'à 6 atomes de carbone.

5. Composé suivant la revendication 1, dans lequel :
R représente un groupe méthyle, éthyle, isopropyle, tertio-butyle, cyclopropyle ou 1-méthylcyclopropyle ;
R¹ représente du chlore, du brome, du fluor, un groupe trifluorométhyle ou méthoxy ;
R² représente de l'hydrogène, du chlore ou un groupe méthoxy ;
R³ représente de l'hydrogène ;
R⁴ est un groupe méthyle ou éthyle ; et
n a la valeur 0, 1 ou 2.

6. Composé suivant la revendication 1, qui est :
la 1-[2-chloro-4-(méthylsulfonyl)phényl]-2-cyano-4,4-diméthylpentane-1,3-dione,
la 1-[2-chloro-4-(méthylsulfonyl)phényl]-2-cyano-3-cyclopropylpropane-1,3-dione,
la 2-cyano-3-cyclopropyl-1-[4-(méthylsulfonyl)-2-trifluorométhylphényl]propane-1,3-dione,
la 1-[2-chloro-4-(méthylsulfonyl)phényl]-2-cyanopentane-1,3-dione,
la 1-[2-chloro-4-(méthylsulfonyl)phényl]-2-cyano-4-méthylhexane-1,3-dione,
la 1-[2-chloro-4-(méthylsulfonyl)phényl]2-cyanohexane-1,3-dione,
la 2-cyano-4,4-diméthyl-1-[4-(méthylsulfonyl)-2-trifluorométhylphényl]pentane-1,3-dione,
la 2-cyano-1-[4-(méthylsulfonyl)-2-trifluorométhylphényl]-3-(1-méthylcyclopropyl)propane-1,3-dione,
la 2-cyano-1-[4-(méthylsulfonyl)-2-trifluorométhylphényl]butane-1,3-dione,
la 1-(2-chloro-4-(méthylsulfényl)phényl)-2-cyano-3-cyclopropylpropane-1,3-dione,
la 2-cyano-3-cyclopropyl-1-(2-fluoro-4-(méthylsulfonyl)phényl)propane-1,3-dione,
la 1-[2-chloro-4-(éthylsulfonyl)phényl]-2-cyano-3-cyclopropylpropane-1,3-dione,
la 2-cyano-3-cyclopropyl-1-[4-(méthylsulfényl)-2-trifluorométhylphényl]-propane-1,3-dione,
la 2-cyano-3-cyclopropyl-1-[2-(méthoxy-4-(méthylsulfényl)phényl]propane-1,3-dione,
la 1-[2-bromo-3-méthoxy-4-(méthylsulfonyl)phényl]-2-cyanobutane-1,3-dione,
la 1-[2,3-dichloro-4-(méthylsulfonyl)-phényl]-2-cyano-3-(1-méthylcyclopropyl)propane-1,3-dione,
la 1-[2-chloro-3-méthoxy-4-(méthylsulfonyl)phényl]-2-cyano-3-cyclopropylpropane-1,3-dione,
la 1-[2-chloro-3-méthoxy-4-(méthylsulfonyl)phényl]-2-cyanobutane-1,3-dione,
la 1-[2-bromo-4-(méthylsulfonyl)phényl]-2-cyano-3-cyclopropylpropane-1,3-dione,
la 1-[2-bromo-4-(méthylsulfonyl)phényl]-2-cyano-3-(1-méthylcyclopropyl)propane-1,3-dione,
la 2-cyano-3-(1-méthylcyclopropyl)-1-[4-(éthylsulfonyl)-2-trifluorométhylphényl]propane-1,3-dione, ou
la 1-[2-chloro-3-méthoxy-4-(méthylsulfonyl)phényl]-2-cyano-3-(1-méthylcyclopropyl)propane-1,3-dione,
ou un sel acceptable en agriculture d'une forme tautomérique énolique de ce composé.

7. Procédé de production d'un composé suivant la revendication 1, qui comprend :
a) la réaction d'un bêta-cétonitrile de formule II avec un chlorure de benzoyle de formule III dans laquelle n a la valeur 0 ou 2 : 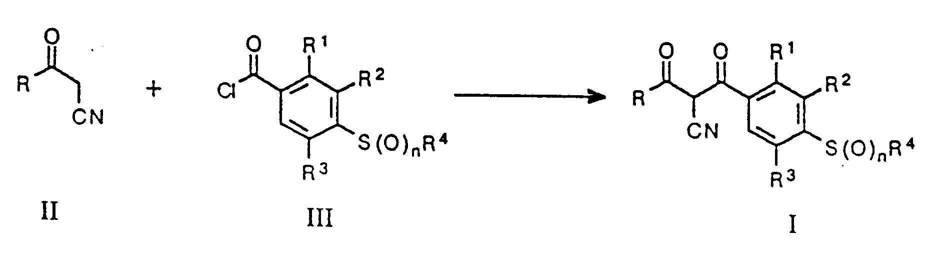 où R, R¹, R², R³ et R⁴ sont tels que définis dans la revendication 1 et n a la valeur 0 ou 2 ;
b) la réaction d'un chlorure d'acide de formule IV avec un bêta-cétonitrile de formule V dans laquelle n a la valeur 0 ou 2 : 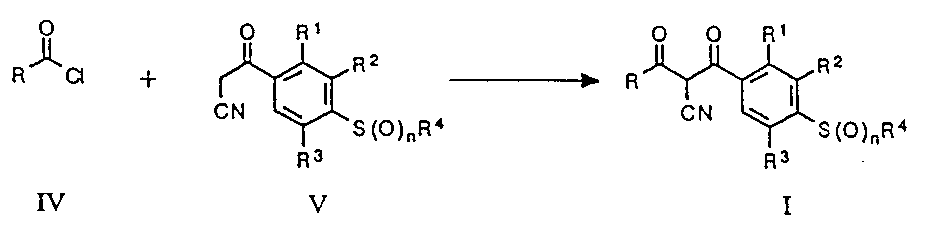 où R, R¹, R², R³ et R⁴ sont tels que définis dans la revendication 1 ; ou
c) l'oxydation facultative d'un composé de formule I dans laquelle n est égal à 0 en un composé dans lequel n a la valeur 1 ou 2 ;
et la transformation facultative d'une 2-cyano-1,3-dione de formule générale I ainsi obtenue en l'un de ses sels acceptables en agriculture.

8. Procédé suivant la revendication 7, dans lequel la réaction est conduite en présence d'une base dans un solvant ou dans un mélange de solvants à une température allant de 0°C au point d'ébullition du solvant.

9. Composition herbicide, qui comprend comme ingrédient actif une quantité à effet herbicide d'un dérivé de 2-cyano-1,3-dione de formule générale I suivant l'une quelconque des revendications 1 à 6 ou d'un sel acceptable en agriculture de ce dérivé et un diluant ou support acceptable du point de vue herbicide, et à titre facultatif un ou plusieurs agents tensio-actifs.

10. Composition herbicide suivant la revendication 9, qui comprend 0,05 à 90 % en poids d'ingrédient actif.

11. Composition herbicide suivant la revendication 9 ou 10, qui comprend 0,05 % à 25 % en poids d'agent tensio-actif et à titre facultatif, un adhésif, un colloïde protecteur, un agent épaississant, un agent de pénétration, un agent stabilisant, un agent séquestrant, un agent anti-agglomération, un agent colorant et/ou un inhibiteur de corrosion.

12. Composition herbicide suivant la revendication 9, 10 ou 11, qui est sous la forme d'un concentré en suspension aqueuse, d'une poudre mouillable, d'une poudre soluble, d'un concentré liquide hydrosoluble, d'un concentré liquide en suspension émulsionnable, de granulés ou d'un concentré émulsionnable.

13. Procédé pour combattre la croissance de mauvaises herbes en un lieu, qui comprend l'application à ce lieu d'un dérivé de 2-cyano-1,3-dione de formule générale I suivant l'une quelconque des revendications 1 à 6 ou d'un sel acceptable en agriculture de ce dérivé.

14. Procédé suivant la revendication 13, dans lequel le lieu est une aire utilisée, ou devant être utilisée, pour des cultures, utilisant un taux d'application compris entre 0,01 kg et 4,0 kg de substance active par hectare.

15. Procédé suivant la revendication 13, dans lequel le lieu n'est pas une aire de culture, utilisant un taux d'application compris entre 1,0 kg et 20,0 kg d'ingrédient actif par hectare.
